(19) Europäisches Patentamt
European Patent Office
Office européen des brevets

(11) Veröffentlichungsnummer: **0 175 364**
**B1**

(12) **EUROPÄISCHE PATENTSCHRIFT**

(45) Veröffentlichungstag der Patentschrift: **06.06.90**

(21) Anmeldenummer: **85111841.4**

(22) Anmeldetag: **19.09.85**

(51) Int. Cl.[5]: **C 07 D 241/06,** C 07 C 255/42, C 07 D 243/08

# (54) Verfahren zur Herstellung von Alpha, Beta-Diaminoacrylnitrilen.

(30) Priorität: **20.09.84 DE 3434525**

(43) Veröffentlichungstag der Anmeldung: **26.03.86 Patentblatt 86/13**

(45) Bekanntmachung des Hinweises auf die Patenterteilung: **06.06.90 Patentblatt 90/23**

(84) Benannte Vertragsstaaten: **CH DE FR GB IT LI**

(56) Entgegenhaltungen:
**SYNTHESIS, Januar 1974, Seiten 34-36; J.A. DEYRUP et al.: "Synthesis of N-substituted diiminosuccinonitrile (DISN) and diaminomaleonitrile (DAMN) derivatives"**

(73) Patentinhaber: **BASF Aktiengesellschaft
Carl-Bosch-Strasse 38
D-6700 Ludwigshafen (DE)**

(72) Erfinder: **Hickmann, Eckhard, Dr.
Kantstrasse 23
D-6701 Dannstadt-Schauernheim (DE)**

**EP 0 175 364 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft eine neues Verfahren zur Herstellung von α,β-Diaminoacrylnitrilen der allgemeinen Formel I

$$R^3R^2NC(R^1)=C(NR^2R^3)CN \qquad I$$

in welcher $R^1$, $R^2$ und $R^3$ gleich oder verschieden und voneinander unabhängig Wasserstoff oder ein Alkyl-, Cycloalkyl-, Aralkyl-, Aryl- oder ein heterocyclischer Rest ist, wobei die organischen Reste $R^2$ auch unter Ausbildung eines 6- oder 7-gliedrigen Ringes oder $R^2$ und $R^3$ unter Ausbildung eines 5- oder 6-gliedrigen Ringes miteinander verbunden sein können.

Es ist bekannt, daß sich 2,3-Di-tert.-butylamino- und 2,3-Di-tert.-butylamino-3-phenyl-crotononitril durch Zersetzung von Nickel-Isocyanid-Komplexen herstellen lassen (Chem. Lett. 939, 1972). Die geringe Ausbeute dieser Reaktion von nur 5 bis 20% läßt eine präparative Anwendung jedoch nicht zu.

Nach H. Böhme und K. H. Weisel (Chem. Ber. *109*, 1908 (1976)) können 3-Arylamino-2-halogen-crotononitrile durch Austausch von Halogen gegen eine Dialkylaminogruppe in die α,β-Diaminocrotononitrile überführt werden. Nach dieser Methode lassen sich nur solche α,β-Diaminoacrylnitrile herstellen, deren β-Aminogruppe arylsubstituiert ist und in denen $R^1$ = Methyl ist.

In Synthesis 35, 1974 wird ferner beschrieben, daß sich α,β-Diaminoacrylnitrile durch Umsetzung von Glyoxaldiimminen mit Acetoncyanhydrin herstellen lassen. Über diesen Weg wurden Verbindungen mit $R^1$ = H oder p—$NO_2$—$C_6H_4$, $R^2$ = H und $R^3$ = tert.-Butyl oder ortho-Toluidin hergestellt.

Auch diese Methode ist nicht allgemein anwendbar. So erlaubt sie z.B. nicht die Synthese des technisch besonders interessanten, bisher unbekannten 1,4,5,6-Tetrahydro-2-cyanopyrazin (TCP).

Der Erfindung lag daher die Aufgabe zugrunde, ein neues Verfahren zur Herstellung von α,β-Diaminoacrylnitrilen zu finden, das es erlaubt, die Reste $R^1$ bis $R^3$ in weite m Rahmen variieren zu können..

Diese Aufgabe wurde mit einem neuen Verfahren zur Herstellung von α,β-Diaminoacrylnitrilen der allgemeinen Formel I

$$R^3R^2NC(R^1)=C(NR^2R^3)CN \qquad I$$

gelöst, in welcher

$R^1$ für Wasserstoff, aliphatische Reste mit 1 bis 30 Kohlenstoffatomen, cycloaliphatische Reste mit 3 bis 8 Ringgliedern oder für aromatishe, heteroaromatische oder araliphatische Reste mit 7 bis 12 Kohlenstoffatomen steht, wobei diese Reste auch substituiert sein können;

$R^2$ und $R^3$ gleich oder verschieden sind und Wasserstoff oder einen aliphatischen Rest mit 1 bis 15 Kohlenstoffatomen, einen cycloaliphatischen Rest mit 3 bis 8 Ringgliedern oder einen aromatischen, heterocyclischen oder araliphatischen Rest mit 7 bis 12 Kohlenstoffatomen bedeuten und wobei die Reste $R^2$ und $R^3$ auch gemeinsam zu einem 5- bis 6-gliedrigen Ring verbunden sein können, der noch ein weiteres Heteroatom tragen kann, dadurch gekennzeichnet, daß man die Komponente A) mit den Komponenten B), C) und D), das sind

A) eine α-Dicarbonylverbindung der allgemeinen Formel $R^1COCHO$, in de $R^1$ die o.a. Bedeutung hat oder ein reaktionsfähiges Derivat dieser Carbonylverbindung;

B) ein Salz de schwefligen Säure oder Schwefeldioxid ode ein Schwefeldioxid lieferndes Reagenz;

C) ein Amin der allgemeinen Formel $HNR^2R^3$, oder ein aliphatisches Diamin der allgemeinen Formel $R^3HN—(CH_2)_n—NHR^3$, in dem die Alkylenkette noch einen Substituenten $R^1$ tragen kann, wobei $R^1$ und $R^3$ die o.a. Bedeutung haben und n die Zahlen 2 oder 3 bedeutet, oder Derivate, die unter den Reaktionsbedingungen in das Amin bzw. Diamin übergehen;

D) flüssige oder gasförmige Blausäure oder ein Blausäure lieferndes Reagenz in beliebiger Reihenfolge umsetzt.

Für die Komination A) + B) + C) + D) ergibt sich folgendes Reaktionsschema:

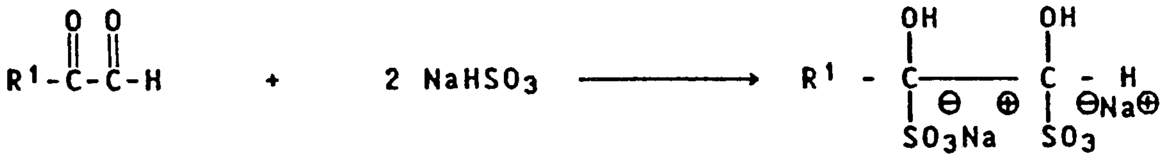

A B II

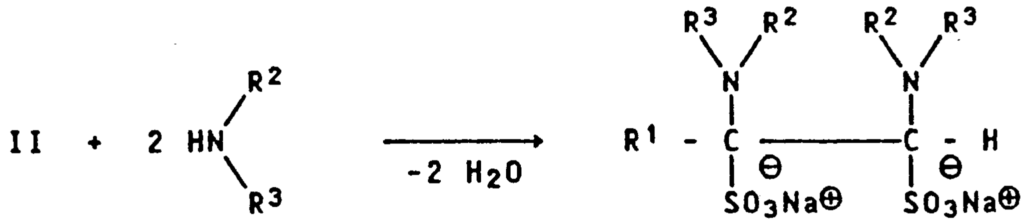


C III

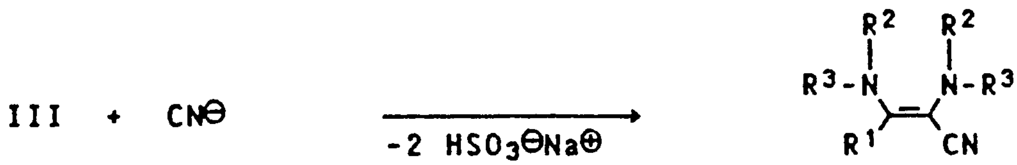


D I

Die Reihenfolge des Zusammengebens der Komponenten A)—D) ist beliebig; man kann auch 2 oder mehr Komponenten gleichzeitig vereinigen oder aber das Gemisch von 2 oder mehr Komponenten mit den übrigen in beliebiger Weise umsetzen. Die Synthese von I läßt sich im "Eintopfverfahren" durchführen, mann kann aber auch jede Zwischenstufe z.B. die Dinatriumsalze der Bis-(α-hydroxysulfonsäure) II oder der Bis-(α-aminosulfonsäure) III isolieren und weiter mit dem(n) noch fehlenden Reaktionspartner(n) zu I umsetzen.

Das Gelingen dieses Verfahrens ist überraschend, da man weiß, daß die Komponenten A), B) und C) in der Wärme miteinander reagieren können, und zwar zu Oxindolen bei Verwendung von sekundären Aminen oder zu offenkettigen bzw. cyclischen Glycinamiden, ausgehend von Monoaminen bzw. von α,β- oder α,γ-Diaminen (vgl. Hinsberg-Oxindol-Synthese, Merck Index 10th ed 1983, Seite ONR-43).

Das Mengenverhältnis der Komponenten A) bis D) kann in weiten Grenzen variiert werden. Aus Gründen der Wirtschaftlichkeit wird man in der Regel nicht allzu weit von einem molaren Verhältnis abweichen, also A:B:C:D ≈ 1:2:2/1 (Monoamin/Diamin):1.

Oft ist es jedoch vorteilhaft, B) im Überschuß einzusetzen, z.B. im Verhältnis A:B = 1:2 bis 4. Auch C) läßt sich in vielen Fällen vorteilhaft im Überschuß verwenden, z.B. A:C = 1:2 bis 5/10 (Monoamin/Diamin). Selbst ein Überschuß an D), z.B. A:D = 1:1 bis 3 ist möglich, ohne Ausbeuteverluste an Wertprodukt I hinnehmen zu müssen. Dieser Befund ist unerwartet; denn in Analogie zur Strecker schen Aminonitrilsynthese aus Monocarbonylverbindungen in der Modifizierung von Bucherer und Knoevenagel (vgl. The Merck Index, 10th ed. 1983, Seite ONR-87) hätte man angenommen, daß die Umsetzung von α-Dicarbonylverbindungen ($R^1COCHO$) mit Aminen ($HNR^2R^3$) nach Strecker zu Bis-(α-aminonitrilen) der allgemeinen Formel $R^3R^2NCR^1(CN)\text{-}CH(CN)NR^2R^3$ geführt hätte.

Nach dem erfindungsgemäßen Verfahren werden α-Dicarbonylverbindungen der allgemeinen Formel $R^1COCHO$ verwendet. Als Beispiele für $R^1$ sind Wasserstoff, aliphatische verzweigte oder unverzweigte Reste mit 1 bis 30, vorzugswise 1 bis 10, insbesondere 1 bis 5 C-Atomen, cycloaliphatische Reste mit 3 bis 8 Ringgliedern, aromatische oder heteroaromatische Reste oder araliphatische Reste mit 7 bis 12 C-Atomen zu nennen.

Die α-Dicarbonylverbindung läßt sich direkt oder in Form eines reaktionsfähigen Derivates, z.B eines Oligo- oder Polymeren, eines Hydrats eines Halbacetals, eines Acetals, eines Enolesters, eines Aminals oder eines Diimins einsetzen, Vorteilhaft wird sie in Form eines der unter den Reaktionsbedingungen auftretenden Zwischenprodukte umgesetzt, z.B. als Bisulfitaddukt, als Cyanhydrin, als Bis-(α-aminosulfonsäure) oder eines ihrer Salze, als offenkettiges oder cyclisches Diimin. Neben den Glyoxalderivaten läßt sich Glyoxal selber besonders vorteilhaft verwenden.

Als Komponente B) läßt sich gasförmiges oder flüssiges Schwefeldioxid verwenden oder vor allem ein Salz der schwefligen Säure, das auch in situ aus anderen Salzen, z.B. aus Disulfiten oder aus

Schwefeldioxid und Base erzeugt werden kann. Als Salz der schwefligen Säure kommen Alkalimetalldisulfit, -sulfit und insbesondere -hydrogensulfit, z.B. Natriumhydrogensulfit in Frage.

Als Komponente C) kommen Amine der allgemeinen Formel $R^2R^3NH$ in Betracht, in der $R^2$ und $R^3$ gleich oder verschieden sind und ein Wasserstoff, ein aliphatischer verzweigter oder unverzweigter Rest mit 1 bis 15, vorzugsweise 1 bis 10, insbesondere 1 bis 5 C-Atomen, ein cycloaliphatischer Rest mit 3 bis 8 Ringgliedern, ein aromatischer oder heterocyclischer Rest oder ein araliphatischer Rest mit 7 bis 12 C-Atomen sein kann, also z.B. Methyl-, Ethyl-, Propyl-, Isopropyl-, Butyl-, tert.Butyl-, Cyclohexyl-, Benzylamin oder Anilin sowie Dimethyl-, Diethyl-, Diisopropylamin oder Methylanilin.

Die Reste $R^2$ und $R^3$ können auch gemeinsam zu einem 5- oder 6-gliedrigen Ring verbunden sein, der noch ein weiteres Heteroatom tragen kann, z.B. Piperidin, Pyrrolidin oder Morpholin. Im Hinblick auf die Synthese der 1,4,5,6-Tetrahydro-2-cyanopyrazine der Formel IV

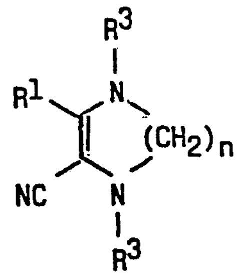

IV

(n = 2, $R^1$ und $R^3$ = H) besonders wichtig sind aliphatische Diamine mit 2 Kohlenstoffatomen in der Alkylenkette, z.B. Ethylendiamin. Die Alkylenkette kann gegebenenfalls noch einen Substituenten $R^1$ tragen, wie z.B. im Propan-1,2-diamin. Außer für Wasserstoff können $R^1$ und $R^3$ in Formel IV für einen Alkyl-, Cycloalkyl-, Aralkyl-, Aryl- oder einen heterocyclischen Rest, der Index n in Formel IV weiterhin für die Zahl 3 stehen.

Die Amine werden in der Regel direkt umgesetzt, können aber auch in Form ihrer Salze, Komplexverbindungen oder verseifbaren Derivate verwendet werden.

Nach dem erfindungsgemäßen Verfahren werden in der Regel α,β-Diaminoacrylnitrile mit zwei gleichen Aminogruppen hergestellt, es ist aber auch möglich, solche mit verschiedenen Aminsubstituenten, in denen sich die beiden Reste $R^2$ und/oder beide Reste $R^3$ unterscheiden, zu synthetisieren.

Als Komponente D) lassen sich flüssige oder gasförmige Blausäure oder ein Blausäure lieferndes Reagenz verwenden. Als geeignete Blausäure liefernde Reagenzien kommen die folgenden in Betracht: a) eine dissoziationsfähige Komplexverbindung, b) ein Addukt der Blausäure, z.B. ein Cyanhydrin, vorteilhaft das Cyanhydrin der zu verwendenden α-Dicarbonylverbindung, c) eine unter Bildung von Blausäure oder Cyanid verlaufende Solvolyse eines Blausäurederivates, z.B. eines Säurecyanids oder eines Silylcyanids und insbesondere d) ein Salz, z.B. Natriumcyanid.

Die Umsetzung der Komponenten A) bis D) kann ohne Lösungsmittel bzw. im Überschuß einer der Komponenten oder in einem Gemisch davon als Lösungsmittel erfolgen. Vorteilhaft wird jedoch in einem Lösungsmittel oder Lösungsmittelgemisch gearbeitet. Hier bieten sich Alkohole, Ketone, Ester, Nitrile, Amide und andere polar-aprotische Lösungsmittel an, z.B. Dimethylformamid, Tetramethylharnstoff und Dimethylsulfoxid. Besonders geeignet ist Wasser als alleiniges Lösungsmittel oder als Hauptkomponente eines Lösungsmittelgemisches.

In der bevorzugten Ausführungsform werden die α,β-Diaminoacrylnitrile ohne Isolierung von Zwischenstufen hergestellt. Man kann aber auch an jeder beliebigen Stelle eine Zwischenstufe isolieren, z.B. das aus A) und B) gebildete Bisulfitaddukt bzw. die Bis-(α-aminosulfonsäure) oder die aus A), B) und C) entstandene Bis-(α-aminosulfonsäure) oder ein Salz davon, gewöhnlich Kristallwasser enthaltend, und diese unter geeigneten Reaktionsbedingungen weiter umsetzen. Diese Arbeitsweise bietet sich dann an, wenn ein Lösungsmittelwechsel im Verlauf der Umsetzung der Reaktionspartner A), B), C) und D) von Vorteil ist.

Häufig wirkt die Zugabe einer Base, z.B. Natronlauge oder eines Amins, insbesondere des eingesetzten Amins oder Diamins in stöchiometrischem Überschuß während der Reaktion oder vor dem Aufarbeiten deutlich ausbeutesteigernd.

Die Umsetzung kann mit Unterdruck, Überdruck oder drucklos, diskontinuierlich oder kontinuierlich erfolgen.

Im allgemeinen werden möglichst milde Temperaturbedingungen gewählt. Die bevorzugte Reaktionstemperatur liegt im Bereich von 0 bis 100, insbesondere 0 bis 50°C. Um einen gebildeten Niederschlag nach Umsetzung der Komponenten A), B) und C) ganz oder weitgehend aufzulösen, empfiehlt es sich in der Regel, das Reaktionsgemisch nach Zugabe der letzten Komponente kurzzeitig auf 40 bis 80°C zu erwärmen. Der Reaktionspartner D) wird vorteilhaft bei niedrigeren Temperaturen, z.B. 0 bis 30°C zugegeben.

Die Isolierung des Wertproduktes I geschieht nach den dafür üblichen Techniken, z.B. durch

Sedimentieren, Hydrozyklonieren, Filtrieren, Zentrifugieren und insbesondere Extrahieren.

Zum Extrahieren eignen sich alle organischen Lösungsmittel, die, zumindest in Gegenwart von Salzen, in Wasser nur beschränkt löslich sind. Besonders geeignet sind beschränkt wasserlösliche organische Lösungsmittel, wobei der pH-Wert der Reaktionslösung vor oder während der Extraktion durch Zugabe einer Base erhöht werden kann die polare Gruppen tragen, z.B. Alkohole ab 4 C-Atomen, Dialkylether, Arylakylether, Ketone ab 4 C-Atomen, Carbonsäureester, Carbonsäuren ab 5 C-Atomen, Nitrile ab 4 C-Atomen, Amide ab 5 C-Atomen. Das Extraktionsmittel wird in der Regel erst nach beendeter Reaktions zugesetzt, man kann es jedoch auch während der Reaktion zugeben oder ganz am Anfang vorlegen.

Die nach dem erfindungsgemäßen Verfahren herstellbaren α,β-Diaminoacrylnitrile eignen sich u.a. als Zwischenprodukte für die Herstellung von Pharmazeutika, Pflanzenschutzmitteln, Farbstoffen und Hilfsmitteln. Sie können, sofern einer der Reste $R^2$ oder $R^3$ Wasserstoff bedeutet, dehydriert werden. Cyclische, sechsgliedrige α,β-Diaminoacrylnitrile liefern dabei Pyrazinderivate. So erhält man z.B. durch Behandeln von 1,4,5,6-Tetrahydro-2-cyanopyrazin mit "Nickelperoxid" Pyrazincarboxamid, das als Komponente in antituberkulös wirksamen Kombinationspräparaten verwendet wird.

In den Beispielen verhalten sich Teile zu Volumenteile wie Kilogramm zu Liter.

## Beispiel 1

Man legt 1210 Teile 38 %ige $NaHSO_3$-Lösung vor, dosiert bei 20 bis 40°C 290 Teile 40 %ige Glyoxallösung zu, gibt bei 20 bis 30°C 120 Teile Ethylendiamin zu und erwärmt 30 min auf ca. 50°C. Zu der auf 0 bis 5°C gekühlten Lösung läßt man 406 Teile 30 %ige wäßrige NaCN-Lösung zulaufen und rührt noch eine Stunde nach. Anschließend setzt man ca. 150 ml 50 %ige Natronlauge zu, wobei sich das Reaktionsgemisch auf ca. 45°C erwärmt. Nach der zweimaligen Extraktion mit je 2000 Volumenteilen Methylethylketon und Eindampfen der vereinigten Extrake erhält man 186 Teile dünnschichtchromatographisch reines 1,4,5,6-Tetrahydro-2-cyanopyrazin (TCP) = 85% d.Th., das nach Umkristallisieren aus Methanol bei 96 bis 98°C schmilzt.

Gefundene Werte der Elementaranalyse des Produktes: C: 54,8%; H: 6,5%; N: 38,7%; Berechnet für $C_5H_7N_3$: C: 55,0%; H: 6,5%; N: 38,5%.

Das Massenspektrum des Produktes weist den Molpeak bei m/e = 109 auf. Das IR-Spektrum eines KBr-Preßlings der Substanz zeigt u.a. folgende charakteristische Bandenmaxima (in $cm^{-1}$): 3276 (s), 3216 (m), 3060 (w), 2719 (s), 1622 (s), 1517 (m), 1343 (m), 1268 (m), 858 (m), 809 (m).

Im NMR-Spektrum (verdünnte Lösung in DDMSO, TMS als innerer Standard, Verschiebungd in ppm) treten folgende Banden auf: ca. 2,8 (Multiplett, 2 H), ca. 3,0 (Multiplett, 2 H), ca. 3,6 (verbreitertes Triplett, 1 H), ca. 6,1 (breites Multiplett, 1 H), ca. 6,55 (Dublett, 1 H).

## Beispiel 2

Man setzt 275 Teile 38 %ige $NaHSO_3$-Lösung, 72,5 Teile 40 %ige Glyoxallösung, 30 Teile Ethylendiamin und 106 Teile 30 %ige wäßrige NaCN-Lösung nach Beispiel 1 um, unterläßt jedoch die Behandlung mit Natronlauge und extrahiert das Reaktionsprodukt kontinuierlich 8 h mit Methyl-t-butylether. Man erhält nach Abtrennen und Eindampfen der organischen Phase 24 Teile = 44% d.Th. analysenreines 1,4,5,6-Tetrahydro-2-cyanopyrazin.

## Beispiel 3

Man setzt 68,75 Teile 38 %ige $NaHSO_3$-Lösung, 14,5 Teile 40 %ige Glyoxallösung, 6 Teile Ethylendiamin und 45,4 Teile 30 %ige wäßrige NaCN-Lösung und 8 Teile 50 %ige Natronlauge nach Beispiel 1 um. Nach Extraktion mit 300 und anschließend 150 Volumenteilen Methylethylketon und Eindampfen der vereinigten Extrakte erhält man 88,3 Teile = 81 % d.Th. dünnschichtchromatographisch reines 1,4,5,6-Tetrahydro-2-cyanopyrazin.

## Beispiel 4

Man legt 55 Teile 38 %ige $NaHSO_3$-Lösung vor, gibt bei 20 bis 40°C 14,5 Teile 40 %ige wäßrige Glyoxallösung zu, rührt ca. 1/2 h nach, kühlt auf 0°C ab, fügt 22,7 Teile 30 %ige wäßrige NaCN-Lösung zu, rührt wieder ca. 1/2 h nach, gibt 6 Teile Ethylendiamin bei 0 bis 14°C zu, erwärmt kurz auf 50°C, kühlt auf Raumtemperatur ab, rührt ca. 2 h nach, setzt 16 Teile 50 %ige wäßrige Natronlauge zu, extrahiert 2 × mit je 300 Volumenteilen Methylethylketon und dampft die vereinigten Extrakte ein. Man erhält 7,0 Teile = 64% d.Th. dünnschichtchromatographisch reines 1,4,5,6-Tetrahydro-2-cyanopyrazin.

## Beispiel 5

Man legt 55 Teile 38 %ige $NaHSO_3$-Lösung bei 0°C vor, gibt bei 0 bis 10°C 22,7 Teile 30 %ige wäßrige NaCN-Lösung zu, rührt 15 min nach, fügt bei 0 bis 16°C 6 Teile Ethylendiamin zu, rührt wieder 15 min nach, gibt bei 0 bis 10°C 14,5 Teiel 40 %ige wäßrige Glyoxallösung zu, erwärmt kurz auf ca. 50°C, rührt noch ca. 2 h bei Raumtemperatur nach, versetzt mit 16 Teilen 50 %iger wäßriger Natronlauge und extrahiert wie bei Beispiel 4 beschrieben. Man erhält 6,3 Teile = 58% d.Th. dünnschichtchromatographisch reines 1,4,5,6-Tetrahydro-2-cyanopyrazin.

Beispiel 6

Man legt 6 Teile Ethylendiamin vor, fügt 55 Teile 38 %ige wäßrige $NaHSO_3$-Lösung bei Raumtemperatur zu, rührt 15 min nach, setzt 14,5 Teile 40 %ige wäßrige Glyoxallösung zu, rührt wieder 15 min nach, versetzt bei 0 bis 12°C mit 22,7 Teilen 30 %iger wäßriger NaCN-Lösung, erwärmt kurz auf 50°C, rührt bei Raumtemperatur 2 h nach, gibt 16 Teile 50 %ige wäßrige Natronlauge zu und arbeitet wie unter Beispiel 4 beschrieben auf. Man erhält 7,8 Teile = 72% d.Th dünnschichtchromatographisch reines 1,4,5,6-Tetrahydro-2-cyanopyrazin.

Beispiel 7

Man legt 14,5 Teile 40 %ige wärige Glyoxallösung vor, gibt bei 0 bis 10°C 6 Teile Ethylendiamin zu, rührt 15 min nach, fügt bei 5 bis 10°C 55 Teile 38 %ige $NaHSO_3$-Lösung zu, versetzt bei −5 bis 0°C mit 22,7 Teilen 30 %iger wäßriger NaCN-Lösung, erwärmt kurz auf 50°C, rührt bei Raumtemperatur 2 h nach, gibt 16 Teile 50% ige wäßrige Natronlauge zu und arbeitet wie unter Beispiel 4 beschrieben auf. Man erhält 4,9 Teile =45% d.Th. dünnschichtchromatographisch reines 1,4,5,6-Tetrahydro-2-cyanopyrazin.

Beispiel 8

Man legt 300 Volumenteile Methylethylketon vor, fügt 55 Teile 38 %ige wäßrige $NaHSO_3$-Lösung zu, gibt bei 20 bis 30°C 14,5 Teile 40 %ige wäßrige Glyoxallösung zu, rührt 15 min nach, setzt bei 20 bis 40°C 6 Teile Ethylendiamin zu, rührt 30 min nach, kühlt auf 0°C ab, gibt 5,1 Teile festes NaCN zu, rührt 10 min nach, erwärmt die Lösung auf 50°C, rührt 2 h nach, setzt 16 Teile 50 %ige wäßrige Natronlauge zu und trennt die organische Phase ab. Man extrahiert die wäßrige Phase mit weiteren 300 Volumenteilen Methylethylketon nach und dampft die vereinigten organischen Phasen ein. Man erhält 8,8 Teile = 81% d.Th. dünnschichtchromatographisch reines 1,4,5,6-Tetrahydro-2-cyanopyrazin.

Beispiel 9

Man legt 275 Teile 38 %ige wäßrige $NaHSO_3$-Lösung vor, gibt bei 20 bis 30°C 72,5 Teile 40 %ige wäßrige Glyoxallösung zu, rührt 30 min nach, fügt bei 15 bis 25°C 30 Teile Ethylendiamin zu und erwämt die Lösung unter Rühren auf ca. 60°C, bis der anfangs gebildete Niederschlag weitgehend in Lösung gegangen ist. Die erhaltene Lösung rührt man in ca. 2000 Volumenteile Methanol ein, saugt den ausgefallenen gelben Niederschlag ab und trocknet ihn im Wasserstrahlvakuum 2 Tage bei ca. 40°C. Man erhält 124 Teile = 75% d.Th. des Dinatriumsalzes der Piperazin-2,3-disulfonsäure als Dihydrat, das folgende Elementarzusammensetzung aufweist: C: 14,5%; H: 3,4%; N: 7,8%; Na: 15,1%; O: 39,7%; S: 19,6%.

Berechnete Zusammensetzung für $C_4H_8N_2Na_2O_6S_2 \cdot 2\,H_2O$: C: 14,9%; H: 3,7%, N: 7,5%; Na: 14,3%; O: 39,8%; S: 19,9%.

Der ermittelte Wassergehalt des Produktes beträgt 11,6% (ber.: 11,2%).

Versetzt man 100 Teile des oben beschriebenen Dinatriumsalzes mit 35 Teilen wäßriger 37 %iger Salzäure, so erhält man nach Absaugen des ausgefallenen Niederschlages, Waschen mit destilliertem Wasser und Trocknen des chloridfreien Rückstandes im Ölpumpenvakuum 37 Teile = 54% d.Th. an Piperazin-2,3-disulfonsäure-monohydrat mit dem Fp. 124 bis 126°C. Das Produkt hat folgende Elementarzusammensetzung: C: 18,1%; H: 4,7%, N: 10,5%; O: 42,5%; S: 24,1%.

Berechnet für $C_4H_{10}N_2O_6S_2 \cdot H_2O$: C: 18,2%; H: 4,6%, N: 10,6%; O: 42,4%; S: 24,2%.

Man legt von dem oben beschriebenen Dinatriumsalz 145 Teile in 450 Volumenteilen Wasser vor, kühlt auf −4°C ab, gibt bei dieser Temperatur die Lösung von 26 Teilen 95 %igem NaCN in 87,5 Volumenteilen Wasser zu, rührt 2 h bei 0°C nach und arbeitet wie bei Beispiel 4 beschrieben auf. Man erhält 16,6 Teile = 34% d.Th. dünnschichtchromatographisch reines 1,4,5,6-Tetrahydro-2-cyanopyrazin.

Beispiel 10

Man legt 72,5 Teile 40 %ige wäßrige Glyoxallösung vor, verdünnt mit ca. 200 Volumenteilen Wasser, leitet bei 20 bis 35°C 64 Teile gasförmiges Schwefeldioxid ein und gibt 30 Teile Ethylendiamin zu, wobei in einer deutlich exothermen Reaktion ein orangefarbener Niederschlag ausfällt. Das Produkt wird abgesaugt, mit Wasser gewaschen und im Wasserstrahlvakuum bei ca. 70°C getrocknet. Man erhält 100 Teile = 71% d.Th. eines gelblichen Produktes (Ethylendiammoniumsalz der 1,2-Dihydroxyethan-1,2-disulfonsäure?) mit der empirischen Elementarzusammensetzung: C: 17,5%; H: 5,2%, N: 10,2%; O: 44,5%; S: 22,0%.

Berechnet für $C_4H_{14}N_2O_8S_2$ C: 17,0%; H: 5,0%, N: 9,9%: O: 45,4%; S: 22,7%.

Neutralisiert man 56,4 Teile des oben beschriebenen Produktes mit 32 Teilen 50 %iger wäßriger Natronlauge und arbeitet analog Beispiel 9 weiter, so erhält man 12,7 Teile = 58% d.Th. dünnschichtchromatographisch reines 1,4,5,6-Tetrahydro-2-cyanopyrazin.

Beispiel 11

Man suspendiert 16,8 Teile des Diimins aus Glyoxal und tert.-Butylamin (1,4-Di-tert.-butyl-1,4-diaza-1,3-butadien) in 50 Volumenteilen Wasser, gibt bei 20 bis 40°C 82,5 Teile 38 %ige wäßrige $NaHSO_3$-Lösung zu, rührt 2 Stunden nach, fügt bei 20 bis 35°C 6 Teile Ethylendiamin zu, rührt 8 h nach, kühlt auf −4°C ab. setzt die Lösung von 5,2 Teilen 95 %igem NaCN in 17,5 Volumenteilen Wasser zu, rührt 8 h nach, stellt den pH durch Zugabe von 50 %iger wäßriger Natronlauge auf 12,4 und arbeitet wie unter Beispiel 4

beschrieben auf. Man erhält 7 Teile = 64% d.Th. dünnschichtchromatographisch reines 1,4,5,6-Tetrahydro-2-cyanopyrazin.

Beispiel 12

Man legt 12 Teile Ethylendiamin in 100 Volumenteilen DMF vor, dosiert bei ca. 20°C 54,2 Teile des Glyoxal-Natriumbisulfit-Adduktes zu, rührt 4 Stunden bei 20 bis 30°C nach, gibt bei ca. 0°C 10,4 Teile 95 %iges NaCN zu, rührt 8 Stunden bei ca. 20°C, filtriert vom Niederschlag ab und destilliert das Lösungsmittel im Ölpumpenvakuum ab. Man erhält 12 Teile = 55% d.Th. eines dunkelgefärbten Rückstandes, der lt. Dünnschichtchromatographie weitgehend aus 1,4,5,6-Tetrahydro-2-cyanopyrazin besteht.

Beispiel 13

Man legt 13,8 Teile Phenylglyoxal und 40 Volumenteile Wasser vor, erwärmt auf ca. 50°C, fügt 61 Teile 38 %ige wäßrige $NaHSO_3$-Lösung und soviel Methanol zu, daß fast vollständige Lösung eintritt. Anschließend gibt man bei ca. 30°C 6 Teile Ethylendiamin zu, erwärmt 30 Minuten auf 50°C, kühlt auf ca. 10°C ab, setzt bei dieser Temperatur die Lösung von 5,2 Teilen 95 %igem NaCN in 13 Volumenteilen Wasser zu, rührt noch 2 h bei Raumtemperatur und arbeitet wie unter Beispiel 4 beschrieben auf. Man erhält 10 Teile = 54% d.Th. 1,4,5,6-Tetrahydro-3-phenyl-2-cyanopyrazin in Form eines rotbraunen, zähen Öles. Dieses Produkt zeigt im Massenspektrum den für die Summenformel $C_{11}H_{11}N_3$ zu erwartenden Molpeak bei e/m = 185 und weist im NMR-Spektrum die für die unterschiedlichen NH-Gruppen typischen Banden bei ca. 3,5 ppm ($N^1$—*H*) bzw. ca. 6,15 ppm ($N^4$—*H*) auf.

Beispiel 14

Man legt 330 Teile 38 %ige wäßrige $NaHSO_3$-Lösung vor, gibt 72,5 Teile 40 %ige wäßrige Glyoxallösung zu, setzt 94 Teile Anilin zu, erwärmt 30 Minuten auf ca. 50°C, kühlt auf Raumtemperatur ab, fügt 80 Teile 50 %ige wäßrige Natronlauge zu und anschließend bei ca. 15°C die Lösung von 26 Teilen 95 %igem NaCN in 60 Volumenteilen Wasser. Man läßt 3 Stunden bei Raumtemperatur nachrühren, extrahiert wie unter Beispiel 4 beschrieben mit Methylethylketon und erhält nach dem Eindampfen der organischen Phase 190 Teile Rohprodukt. Zur weiteren Reinigung wird das Rohprodukt zweimal mit je ca. 300 Volumenteilen Methyl-tert.-butylether extrahiert, die vereinigten Extrakte werden eingedampft. Man erhält 70 Teile = 30% d.Th. α,β-Dianilino-acrylnitril. Das Produkt zeigt im Massenspektrum den für die Summenformel $C_{15}H_{13}N_3$ zu erwartenden Molpeak bei m/e = 235 und weist im NMR-Spektrum die für die unterschiedlichen NH-Gruppen typischen Banden bei ca. 3,35 ppm ($N^1$—*H*) bzw. ca. 5,0 ppm ($N^4$—*H*) auf.

Beispiel 15

Man legt 200 Teile "Nickelperoxid" (frisch aus Nickelsulfathexahydrat und natronlaugehaltiger Natriumhypochloritlösung bereitet und im Vakuum getrocknet) in 600 Volumenteilen Benzol vor, fügt 11 Teile 1,4,5,6-Tetrahydro-2-cyanopyrazin zu und erhitzt 3 Stunden unter Rückfluß. Die fast farblose flüssige Phase wird abfiltriert, und der Filterrückstand wird mit Methanol digeriert. Eindampfen der methanolischen Lösung liefert 2 Teile = 18% d. Th. Pyrazincarboxamid.

**Patentansprüche**

1. Verfahren zur Herstellung von α,β-Diaminoacrylnitrilen der allgemeinen Formel I

$$R^3R^2NC(R^1)=C(NR^2R^3)CN \qquad I$$

in welcher

$R^1$ für Wasserstoff, aliphatische Reste mit 1 bis 30 Kohlenstoffatomen, cycloaliphatische Reste mit 3 bis 8 Ringgliedern oder für aromatishe, heteroaromatische oder araliphatische Reste mit 7 bis 12 Kohlenstoffatomen steht, wobei diese Reste auch substituiert sein können;

$R^2$ und $R^3$ gleich oder verschieden sind und Wasserstoff oder einen aliphatischen Rest mit 1 bis 15 Kohlenstoffatomen, einen cycloaliphatischen Rest mit 3 bis 8 Ringgliedern oder einen aromatischen, heterocyclischen oder araliphatischen Rest mit 7 bis 12 Kohlenstoffatomen bedeuten und wobei die Reste $R^2$ und $R^3$ auch gemeinsam zu einem 5- bis 6-gliedrigen Ring verbunden sein können, der noch ein weiteres Heteroatom tragen kann, dadurch gekennzeichnet, daß man die Komponente A) mit den Komponente B), C) und D), das sind

A) eine α-Dicarbonylverbindung der allgemeinen Formel $R^1COCHO$, in der $R^1$ die o.a. Bedeutung hat oder ein reaktionsfähiges Derivat dieser Carbonylverbindung;

B) ein Salz de schwefligen Säure oder Schwefeldioxid oder ein Schwefeldioxid lieferndes Reagenz;

C) ein Amin der allgemeinen Formel $HNR^2R^3$, oder ein aliphatisches Diamin der allgemeinen Formel $R^3HN—(CH_2)_n—NHR^3$, in dem die Alkylenkette noch einen Substituenten $R^1$ tragen kann, wobei $R^1$ und $R^3$ die o.a. Bedeutung haben und n die Zahlen 2 oder 3 bedeutet, oder Derivate, die unter den Reaktionsbedingungen in das Amin bzw. Diamin übergehen;

D) flüssige oder gasförmige Blausäure oder ein Blausäure lieferndes Reagenz in beliebige Reihenfolge umsetzt.

2. Verfahren nach Anspruch 1, dadurch gekennzeichnet, daß man die α-Dicarbonylverbindung in Form

eines Bilsulfit-Adduktes oder eines Cyanhydrins verwendet.

3. Verfahren nach Anspruch 1, dadurch gekennzeichnet, daß man die α-Dicarbonylverbindung in Form einer Bis-(α-aminosulfonsäure) oder eines ihrer Salze einsetzt.

4. Verfahren nach Anspruch 1, dadurch gekennzeichnet, daß man die α-Dicarbonylverbindung in Form eines offenkettigen oder cyclischen Diimins einsetzt.

5. Verfahren nach Anspruch 1, dadurch gekennzeichnet, daß die Komponente A Glyoxal oder ein Gyoxal-Oligomeres oder -Polymeres oder ein Glyoxalderivat ist.

6. Verfahren nach Anspruch 1, dadurch gekennzeichnet, daß man als Salz der schwefligen Säure ein Alkalimetallhydrogensulfit oder -sulfit oder ein Alkalimetalldisulfit verwendet.

7. Verfahren nach Anspruch 1, dadurch gekennzeichnet, daß man als Aminkomponente Ethylendiamin verwendet.

8. Verfahren nach Anspruch 1, dadurch gekennzeichnet, daß man als Blausäure lieferndes Reagenz ein Alkalicyanid oder ein Cyanhydrin verwendet.

9. Verfahren nach Anspruch 1, dadurch gekennzeichnet, daß man die Umsetzungen in überwiegend wäßriger Lösung durchführt.

10. Verfahren nach Anspruch 1, dadurch gekennzeichnet, daß man die Komponente A) mit den Reaktionspartnern B) und C) bei Temperaturen von 0 bis 100°C umsetzt und die Komponente D) bei Temperaturen von 0 bis 30°C zugibt.

11. Verfahren nach Anspruch 1, dadurch gekennzeichnet, daß man die Reaktion entweder ohne Isolierung von Zwischenstufen durchführt oder eine Zwischenstufe isoliert und sie in einem anderen als dem zuvor verwendeten Lösungsmittel bzw. Lösungsmittelgemisch weiter umsetzt.

12. Verfahren nach Anspruch 1, dadurch gekennzeichnet, daß man das Produkt der allgemeinen Formel I durch Extraktion mit einem polaren, beschränkt wasserlöslichen organischen Lösungsmittel isoliert, wobei der pH-Wert der Reaktionslösung vor oder während der Extraktion durch Zugabe einer Base erhöht wird.

13. Verbindungen der Formel

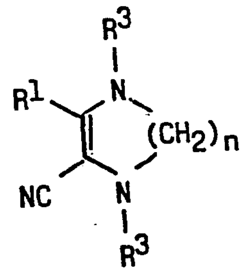

in der n für die Zahlen 2 und 3 steht, und die Reste $R^1$ und $R^2$ gleich oder verschieden und voneinander unabhängig Wasserstoff oder einen Alkyl-, Cycloalkyl-, Aralkyl-, Aryl- oder heterocyclischen Rest gemäß Anspruch 1 bedeuten.

14. Verbindung der Formel

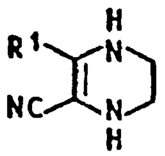

in der $R^1$ Wasserstoff oder Phenyl bedeutet.

**Revendications**

1. Procédé de préparation d'α,β-diaminoacrylonitriles de formule générale I

$$R^3R^2NC(R^1)=C(NR^2R^3)CN \qquad I$$

dans laquelle

$R^1$ est mis pour un atome d'hydrogène, des restes aliphatiques à 1—30 atomes de carbone, des restes cycloaliphatiques à 3—8 chaînons ou pour des restes aromatiques, hétéroaromatiques ou araliphatiques à 7—12 atomes de carbone, ces restes pouvant être également substitués;

$R^2$ et $R^3$ sont identiques ou différents et représentent chacun un atome d'hydrogène ou un reste aliphatique à 1—15 atomes de carbone, un reste cycloaliphatique à 3—8 chaînons ou un reste aromatique, hétérocyclique ou araliphatique à 7—12 atomes de carbone, les restes $R^2$ et $R^3$ pouvant aussi être reliés l'un à l'autre en formant un noyau à 5 ou 6 chaînons qui peut encore porter un hétéroatome supplémentaire,

caractérisé en ce qu'on fait réagir dans n'importe quel ordre le composant A) avec les composants B), C) et D), qui sont

A) un composé α-dicarbonylé de formule générale $R^1COCHO$, dans laquelle $R^1$ a la signification précédente, ou un dérivé réactif de ce composé carbonylé;

B) un sel de l'acide sulfureux ou de l'anhydride sulfureux ou un réactif donnant de l'anhydride sulfureux;

C) une amine de formule générale $HNR^2R^3$ ou une diamine aliphatique de formule générale $R^3HN—(CH_2)_n—NHR^3$, dans laquelle la chaîne alkylène peut encore porter un substituant $R^1$, $R^1$ et $R^3$ ayant les significations précédentes et n étant mis pour le nombre 2 ou 3, ou des dérivés qui se transforment en l'amine ou la diamine dans les conditions de la réaction;

D) de l'acide cyanhydrique liquide ou gazeux ou un réactif donnant de l'acide cyanhydrique.

2. Procédé selon la revendication 1, caractérisé en ce qu'on utilise le composé α-dicarbonylé sous la forme d'un produit d'addition avec un bisulfite ou d'une cyanhydrine.

3. Procédé selon la revendication 1, caractérisé en ce qu'on utilise le composé α-dicarbonylé sous la forme d'un acide bis-(α-aminosulfonique) ou de l'un de ses sels.

4. Procédé selon la revendication 1, caractérisé en ce qu'on utilise le composé α-dicarbonylé sous la forme d'une diimine en chaîne ouverte ou cyclique.

5. Procédé selon la revendication 1, caractérisé en ce que le composant A est le glyoxal, un oligomère ou polymère du glyoxal ou un dérivé du glyoxal.

6. Procédé selon la revendication 1, caractérisé en ce qu'on utilise, comme sel de l'acide sulfureux, un hydrogénosulfite ou sulfite de métal alcalin ou un disulfite de métal alcalin.

7. Procédé selon la revendication 1, caractérisé en ce qu'on utilise de l'éthylènediamine comme composant amine.

8. Procédé selon la revendication 1, caractérisé en ce qu'on utilise, comme réactif donnant de l'acide cyanhydrique, un cyanure alcalin ou une cyanhydrine.

9. Procédé selon la revendication 1, caractérisé en ce qu'on conduit les réactions en solution essentiellement aqueuse.

10. Procédé selon la revendication 1, caractérisé en ce qu'on fait réagir le composant A) avec les partenaires réactionnels B) et C) à des températures de 0 à 100°C et on ajoute le composant D) à des températures de 0 à 30°C.

11. Procédé selon la revendication 1, caractérisé en ce qu'ou bien on conduit la réaction sans isolement de produits intermédiaires, ou bien ou isole unproduit intermédiaire et on le remet en réaction dans un autre solvant ou mélange de solvants que celui qui a été utilisé précédemment.

12. Procédé selon la revendication 1, caractérisé en ce qu'on isole le produit de formule générale I par extraction avec un solvant organique polaire ayant une solubilité limitée dans l'eau, le pH de la solution réactionnelle étant élevé par addition d'une base avant ou pendant l'extraction.

13. Composés de formule

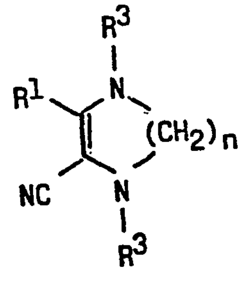

dans laquelle n est mis pour les nombres 2 et 3 et les restes $R^1$ et $R^3$ sont identiques ou différents et représentent, indépendamment l'un de l'autre, des atomes d'hydrogène ou des restes alkyle, cycloalkyle, aralkyle, aryle ou hétérocycliques selon la revendication 1.

14. Composé de formule

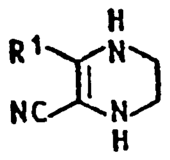

dans laquelle $R^1$ représente un atome d'hydrogène ou un radical phényle.

**Claims**

1. A process for the preparation of an α,β-diaminoacrylonitrile of the general formula I

$$R^3R^2NC(R^1)=C(NR^2R^3)CN \quad I$$

where $R^1$ is hydrogen, an aliphatic radical of 1 to 30 carbon atoms, a cycloaliphatic radical having 3 to 8 ring members or an aromatic, heteroaromatic or araliphatic radical of 7 to 12 carbon atoms, and these radicals may be substituted, and $R^2$ and $R^3$ are identical or different and are each hydrogen, an aliphatic radical of 1 to 15 carbon atoms, a cycloaliphatic radical having 3 to 8 ring members or an aromatic, heterocyclic or araliphatic radical of 7 to 12 carbon atoms, and the radicals $R^2$ and $R^3$ may furthermore be bonded to one another to form a 5-membered or 6-membered ring, which may carry a further hetero atom, wherein component A) is reacted with components B), C) and D), i.e.

A) an α-dicarbonyl compound of the general formula $R^1COCHO$, where $R^1$ has the above meanings, or a reactive derivative of this carbonyl compound,

B) a salt of sulfurous acid or sulfur dioxide or a sulfur dioxide donor,

C) an amine of the general formula $HNR^2R^3$, or an aliphatic diamine of the general formula $R^3HN—(CH_2)_n\text{-}NHR^3$, where the alkylene chain may carry a further substituent $R^1$, $R^1$ and $R^3$ have the above meanings and n is 2 or 3, or a derivative which is converted to the amine or diamine under the reaction conditions, and

D) liquid or gaseous hydrocyanic acid or a hydrocyanic acid donor,

in any sequence.

2. A process as claimed in claim 1, wherein the α-dicarbonyl compound is used in the form of a bisulphite adduct or of a cyanohydrin.

3. A process as claimed in claim 1, wherein the α-dicarbonyl compound is used in the form of a bis-α-aminosulfonic acid or of one of its salts.

4. A process as claimed in claim 1, wherein the α-dicarbonyl compound is used in the form of an open-chain or cyclic diimine.

5. A process as claimed in claim 1, wherein the component A is glyoxal, a glyoxal oligomer or polymer or a glyoxal derivative.

6. A process as claimed in claim 1, wherein an alkali metal bisulfite or sulfite or an alkali metal disulfite is used as the salt of sulfurous acid.

7. A process as claimed in claim 1, wherein the amine component used is ethylene diamine.

8. A process as claimed in claim 1, wherein the hydrocyanic acid donor used is an alkali metal cyanide or a cyanohydrin.

9. A process as claimed in claim 1, wherein the reactions are carried out in predominantly aqueous solution.

10. A process as claimed in claim 1, wherein component A) is reacted with reactants B) and C) at from 0 to 100°C, and component D) is added at from 0 to 30°C.

11. A process as claimed in claim 1, wherein either the reaction is carried out without isolation of intermediates, or an intermediate is isolated and is reacted further in a solvent or solvent mixture other than that used previously.

12. A process as claimed in claim 1, wherein the product of the general formula I is isolated by extraction with a polar organic solvent of limited solubility in water, the pH of the reaction solution being increased, before or during the extraction, by adding a base.

13. A compound of the formula

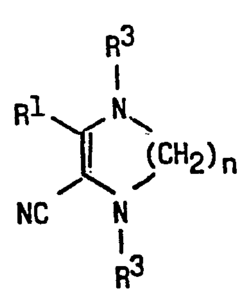

where n is 2 or 3 and $R^1$ and $R^3$ are identical or different and independently of one another are each hydrogen, alkyl, cycloalkyl, aralkyl, aryl or a heterocyclic radical as claimed in claim 1.

14. A compound of the formula

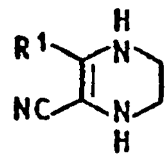

where $R^1$ is hydrogen or phenyl.